# EUROPEAN PATENT SPECIFICATION

(11) **EP 2 600 824 B1**
(45) Date of publication and mention of the grant of the patent: **10.06.2020**
(21) Application number: 11801076.8
(22) Date of filing: 22.06.2011
(51) Int. Cl.: A61K 8/34, A61K 8/42, A61K 8/44, A61K 8/49, A61K 8/60, A61K 8/67, A61K 8/88, A61Q 17/04, A61Q 19/08

(54) **COSMETIC COMPOSITION FOR PREVENTING SKIN AGING**
KOSMETISCHE ZUSAMMENSETZUNG GEGEN HAUTALTERUNG
COMPOSITION COSMÉTIQUE POUR PRÉVENIR LE VIEILLISSEMENT CUTANÉ

(30) Priority: 03.05.2011 KR 20110042071; 29.06.2010 KR 20100061733
(43) Date of publication of application: 12.06.2013
(73) Proprietor: Amorepacific Corporation, Seoul 140-777 (KR)
(72) Inventor: KIM, Hyeon Chung, Yongin-si Gyeonggi-do 446-729 (KR); PARK, Sung Il, Yongin-si Gyeonggi-do 446-729 (KR); KIM, Youn Joon, Yongin-si Gyeonggi-do 446-729 (KR); HAN, Sang Hoon, Yongin-si Gyeonggi-do 446-729 (KR); KIM, Hyun Hee, Yongin-si Gyeonggi-do 446-729 (KR)
(74) Representative: Novagraaf Technologies
(86) International application number: PCT/KR2011/004546
(87) International publication number: WO 2012/002669

(56) References cited:
- EP-A1- 1 262 166
- EP-A1- 1 591 105
- EP-A1- 1 604 643
- EP-A2- 1 595 527
- WO-A1-98/01107
- DE-A1- 10 026 571
- DE-A1- 10 040 932
- DE-A1-102004 049 062
- DE-A1-102008 061 044
- DE-A1-102008 061 045
- JP-A- 2004 189 645
- JP-A- 2004 536 838
- JP-A- 2005 336 111
- JP-A- 2007 509 899
- JP-A- 2009 234 936
- KR-A- 20080 111 798
- SCHROEDER P ET AL: "The role of near infrared radiation in photoaging of the skin", EXPERIMENTAL GERONTOLOGY, ELSEVIER SCIENCE, OXFORD, GB, vol. 43, no. 7, 1 July 2008 (2008-07-01), pages 629-632, XP022832860, ISSN: 0531-5565, DOI: 10.1016/J.EXGER.2008.04.010 [retrieved on 2008-04-27]
- PETER SCHROEDER ET AL: "Infrared Radiation-Induced Matrix Metalloproteinase in Human Skin: Implications for Protection", JOURNAL OF INVESTIGATIVE DERMATOLOGY, vol. 128, no. 10, 1 May 2008 (2008-05-01), pages 2491-2497, XP055215237, ISSN: 0022-202X, DOI: 10.1038/jid.2008.116

## Description

### Technical Field

This disclosure relates to a cosmetic use of a composition for preventing aging caused by infrared (IR) rays.

### Background Art

It is generally known that ultraviolet (UV) rays having a shorter wavelength than visible rays may adversely affect human skin. Thus, many studies have been conducted to develop UV-blocking products.

However, effects of infrared (IR) rays upon skin are not particularly known to date. IR rays occupy 80% of the sunlight, have a lower tendency to reflect or scatter by microparticles in the atmosphere than UV or visible rays, and transmit through the air to reach the ground surface while not being interrupted by oxygen or nitrogen molecules in the air.

Recently, it has been demonstrated that IR rays having a blood circulation-stimulating effect and hyperthermia effect may adversely affect human skin. Particularly, it has been shown that IR rays may stimulate formation of skin wrinkles. The mechanism of skin wrinkle formation caused by IR rays is different from the mechanism of skin wrinkle formation caused by UV rays. Therefore, application of known UV blocking agents onto skin when the skin is exposed to sunlight may not prevent skin aging caused by IR rays.

Therefore, there is a need for developing a formulation for blocking rays over a wide range of wavelengths, the formulation being capable of blocking IR rays as well as UV rays.

Document EP 1 262 166 discloses a cosmetic and/or pharmaceutical composition comprising an extract of Pterocarpus marsupium and dicarboxylic acid extract and its use in combination with amino acids, and mentions that this could have a preventive and curative effect on hair loss and signs of skin aging and revitalizing and reactivating activity on the skin. In particular, this document discloses that the composition comprising extracts of Pterocarpus marsupium, in particular Trichodyn, reduce the effect of UVB radiation on in vitro cultured human keratinocytes. This document is silent about any effect on infrared (IR) rays.

Document EP1 591 105 discloses uses of antioxidants for preparing a pharmaceutical or cosmetic composition for protecting skin against damage by infrared radiation. This document does not disclose any data demonstrating any effect of protection of the skin against damage by infrared radiation.

Schroeder et al.: "The role of near infrared radiation in photoaging of the skin", Experimental gerontology, Elsevier Science, Oxford, GB, vol. 43, no. 7, 1 July 2008, pages 629-632, generally reviews the cutaneous effects of IRA-radiation, the underlying molecular mechanisms and the available protective strategies.

Schroeder et al.: "Infrared radiation-induced matrix metalloproteinase in human skin: implications for protection", Journal of investigative dermatology, vol. 128, no. 10, 1 May 2008, pages 2491-2497, teach that IRA irradiation most likely promotes premature skin aging and that topical application of appropriate antioxidants represents an effective photoprotective strategy.

### Disclosure of Invention

### Technical Problem

This disclosure is directed to providing a cosmetic composition for preventing aging caused by infrared (IR) rays, when applied onto skin.

### Solution to Problem

In one general aspect, there is provided a cosmetic use of a composition including, as an active ingredient, at least one amine selected from the group consisting of alanine, lysine, or a salt thereof in an amount of 0.001-20 wt.% based on the total weight of the composition for blocking near-infrared (IR) rays to prevent skin ageing.

### Advantageous Effects of Invention

The composition for use according to an embodiment of this disclosure may prevent aging by blocking ultraviolet (UV) rays and infrared (IR) rays, and thus may be used widely in the field of beauty and cosmetics.

### Brief Description of Drawings

The above and other aspects, features and advantages of the disclosed exemplary embodiments will be more apparent from the following detailed description taken in conjunction with the accompanying drawings in which:
Figs. 1 to 4 are graphs showing the results of infrared protectabilities measured by Fourier-transform infrared spectroscopy; and
Fig. 5 is a graph showing the results of near-IR protectabilities measured by Fourier-transform near infrared spectroscopy.

### Best Mode for Carrying out the Invention

Exemplary embodiments will be described more fully hereinafter. This disclosure may, however, be embodied in many different forms and should not be construed as limited to the exemplary embodiments set forth therein. Rather, these exemplary embodiments are provided so that this disclosure will be thorough and complete, and will fully convey the scope of this disclosure to those skilled in the art. In the description, details of well-known features and techniques may be omitted to avoid unnecessarily obscuring the presented embodiments.

In one aspect, the composition according to an embodiment of this disclosure is a cosmetic composition for preventing and improving aging, and includes at least one selected from the group consisting of amines, amides and polyols, as an active ingredient.

In one embodiment, the cosmetic composition used is a composition for preventing thermal aging, and more particularly, for blocking heat, or near-infrared (IR) and IR rays. The composition for use may further include an ultraviolet (UV) blocking agent, and prevent thermal aging and photo-aging at the same time. For example, the composition according to this embodiment blocks UV A, UV B, near-IR and IR rays at the same time.

As used herein, the term 'thermal aging' means a phenomena of skin aging caused by heat, and includes skin aging that proceeds when the skin is exposed continuously to a high temperature, as well as skin aging that proceeds when the skin is exposed to IR rays. Thermal aging may be found frequently in those working in places requiring long-term exposure to a high temperature, or staying in Korean dry sauna rooms or sauna baths for a long time. Thermal skin aging may also proceed when the skin is exposed to IR rays of the sunlight. Particularly, IR and near-IR rays have been thought heretofore to have no harmful effect upon skin. However, repeated exposure to IR and near-IR rays causes a rapid drop in synthesis of collagen and an increase in synthesis of matrix metalloproteinase (MMP), which is a collagen-decomposing enzyme, resulting in skin aging.

As used herein, the term 'photo-aging' means skin aging occurring upon the exposure to sunlight, particularly, skin aging caused by the exposure to UV rays. Typical examples of phenomena occurring when photo-aging proceeds include thick and deep wrinkles on the exposed portions, such as faces, necks, arms and hands, and pigment deposition, such as age spots and blemishes. Unlike biological aging including simple skin thinning and loosening, photo-aged skin has materials similar to abnormal elastic fibers packed closely in the upper derma. Thus, the derma may not perform their original function of supporting skin and retaining moisture. UV rays cause direct gene damages in skin cells, leading to skin aging, skin cancers and dermatitis, thereby causing generation of a large amount of active oxygen species secondarily and further aging by oxidation. As a result, this inhibits formation of collagenous and elastic fibers forming the derma and stimulates functions of collagen-decomposing enzymes, leading to a drop in collagenous and elastic fibers.

UV rays mean electromagnetic waves having a shorter wavelength than visible rays, i.e. a wavelength of 397-10 nm, when dispersing the light emitted by the sun through a prism. IR rays are referred to as heat rays while UV rays are also referred to as actinic rays due to their strong chemical actions. In addition, UV rays may be further classified, depending on the magnitude of wavelength, into near-UV (290 nm or more), crystal-range UV (290-190 nm, a wavelength range transmitted through crystal), Schumann rays (190-120 nm), Reimann rays (120-60 nm) and Millikan rays (60 nm or less). UV rays having a wavelength of 190 nm or less may also be referred to as far-UV rays.

IR rays mean electromagnetic waves present at the external portion of the end of the red line, when dispersing the light emitted by the sun through a prism. IR rays are characterized by their stronger thermal action than visible or UV rays. Thus, IR rays may also be referred to as heat rays. IR rays may be classified, depending on the magnitude of wavelength, into near-IR (780-3000 nm), IR (3000-25000 nm) and far-IR (25000 nm or more).

It has been found that the cosmetic composition described herein blocks IR rays (including near-IR and IR rays), which, otherwise, may not be blocked by known UV blocking agents.

In one embodiment, a cosmetic use of a composition including at least one amine selected from the group consisting of alanine, lysine, or a salt thereof in an amount of 0.001-20 wt.% based on the total weight of the composition provides an excellent effect of blocking near-IR and IR rays, as demonstrated by the following test examples.

According to an embodiment, amines may be present in an amount of 1-10 wt%, based on the total weight of the composition. When amines are present in an excessively small amount, the composition provides poor quality as a near-IR and IR absorbing agent, so that it may show an insufficient effect of preventing thermal aging. On the other hand, when amines are present in an excessively large amount, the composition may cause skin irritation or may provide a bad touch in use.

The cosmetic composition described herein prevents thermal aging of skin. Particularly, it is shown that the cosmetic composition described herein prevents or blocks progress of aging caused by the exposure of skin to heat, IR and/or near-IR rays. In one embodiment, the composition for use may further include a UV-blocking agent. According to an embodiment, the composition for use further including a UV-blocking agent prevents thermal aging caused by IR absorption and photo-aging caused by UV absorption at the same time. There is no particular limitation in the UV-blocking agent, as long as it has an effect of blocking UV rays. Such UV-blocking agents may include organic or inorganic UV-blocking agents.

Particular examples of organic UV-blocking agents may include at least one selected from the group consisting of octocrylene, octyl dimethyl para-aminobenzoic acid (PABA), octyl methoxycinnamate, octyl salicylate, octyl triazone, butylmethoxy dibenzoylmethane, benzopheneone, oxybenzone, sulisobenzone, dioxybenzone, Mexoryl-SX, ensulizole, meradimate, avobenzone, isoamyl paramethoxycinnamate, bis-ethylhexyloxyphenol methoxyphenyltriazine and methylene bis-benzotriazolyl tetramethylbutylphenol. Particular examples of inorganic UV-blocking agents may include at least one selected from the group consisting of titanium dioxide, titanium oxide, zinc oxide, boron nitride, cerium oxide and iron oxides. Such organic or inorganic UV-blocking agents may be used with no particular limitation, as long as they do not adversely affect the effect of preventing thermal aging when added to the cosmetic composition described herein.

The cosmetic composition described herein may be formulated in various manners with no particular limitation. For example, the cosmetic composition may be formulated into astringent, nutrient lotion, nutrient cream, massage cream, pack, sun cream, foundation or makeup base. In one embodiment, the cosmetic composition used may be one blocking near-IR and IR rays. In another embodiment, the composition for use may be one blocking UV rays and IR rays at the same time. In still another embodiment, the composition for use may be one blocking UV A (320-400 nm), UV B (280-320 nm), near-IR and IR rays at the same time. Each type of formulation may be provided easily in a manner known to those skilled in the art by selecting various ingredients other than the essential ingredients depending on the intended use.

### Mode for Invention

The examples and experiments will now be described. The following examples and experiments are for illustrative purposes only and not intended to limit the scope of this disclosure.

### [Examples 1 to 11 and Comparative Example]

The cosmetic compositions of Examples 1 to 11 and Comparative Example are provided by using the ingredients as shown in the following Tables 1 and 2.

Examples 1-5 and 8-11 are included for illustrative purposes and are not according to the invention.

### Table 1

**[Table 1]**

| Ingredients | Comp. Ex. | Ex. 1 | Ex. 2 | Ex. 3 | Ex. 4 | Ex. 5 |
|---|---|---|---|---|---|---|
| Deionized water | to 100 | to 100 | to 100 | to 100 | to 100 | to 100 |
| EDTA-2Na | q.s. | q.s. | q.s. | q.s. | q.s. | q.s. |
| Inositol | - | 5 | - | - | - | - |
| Urea | - | - | 5 | - | - | - |
| Niacinamide | - | - | - | 5 | - | - |
| N-acetyl glucosamine | - | - | - | - | 5 | - |
| Polyglutamic acid | - | - | - | - | - | 5 |
| Phenoxyethanol | q.s. | q.s. | q.s. | q.s. | q.s. | q.s. |
| Cetearyl alcohol | 1 | 1 | 1 | 1 | 1 | 1 |
| Phytosqualane | 5 | 5 | 5 | 5 | 5 | 5 |
| Cetearyl alcohol/cetearyl glucoside mixing ratio | 1 | 1 | 1 | 1 | 1 | 1 |
| Tromethamine | q.s. | q.s. | q.s. | q.s. | q.s. | q.s. |
| Carbomer | q.s. | q.s. | q.s. | q.s. | q.s. | q.s. |

### Table 2

**[Table 2]**

| Ingredients | Ex. 6 | Ex. 7 | Ex. 8 | Ex. 9 | Ex. 10 | Ex. 11 |
|---|---|---|---|---|---|---|
| Deionized water | to 100 | to 100 | to 100 | to 100 | to 100 | to 100 |
| EDTA-2Na | q.s. | q.s. | q.s. | q.s. | q.s. | q.s. |
| L-alanine | 5 | - | - | - | - | - |
| L-lysine HCl | - | 5 | - | - | - | - |
| Panthenol | - | - | 5 | - | - | - |
| Erythritol | - | - | - | 5 | - | - |
| Mannitol | - | - | - | - | 5 | - |
| Xylitol | | | | | | 5 |
| Phenoxyethanol | q.s. | q.s. | q.s. | q.s. | q.s. | q.s. |
| Cetearyl alcohol | 1 | 1 | 1 | 1 | 1 | 1 |
| Phytosqualane | 5 | 5 | 5 | 5 | 5 | 5 |
| Cetearyl alcohol/cetearyl glucoside mixing ratio | 1 | 1 | 1 | 1 | 1 | 1 |
| Tromethamine | q.s. | q.s. | q.s. | q.s. | q.s. | q.s. |
| Carbomer | q.s. | q.s. | q.s. | q.s. | q.s. | q.s. |

### [Test Example 1] Determination of IR blocking capability

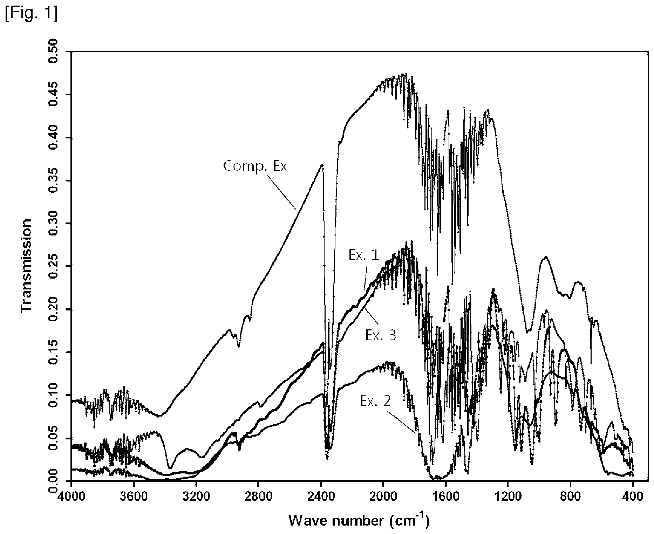

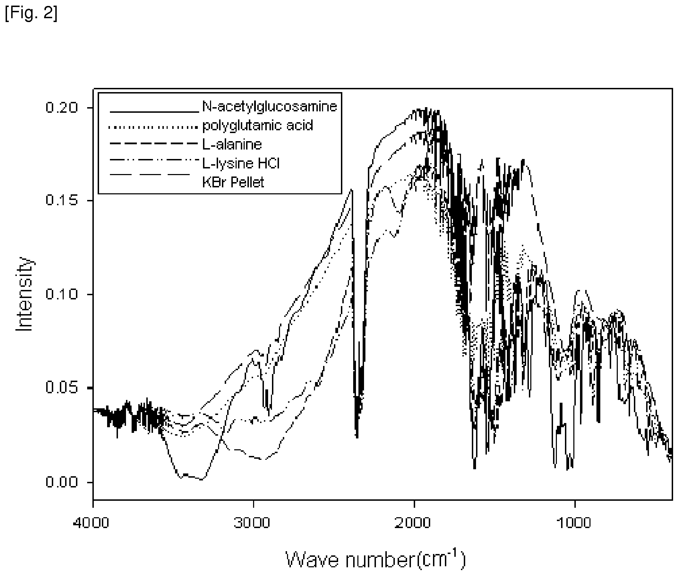

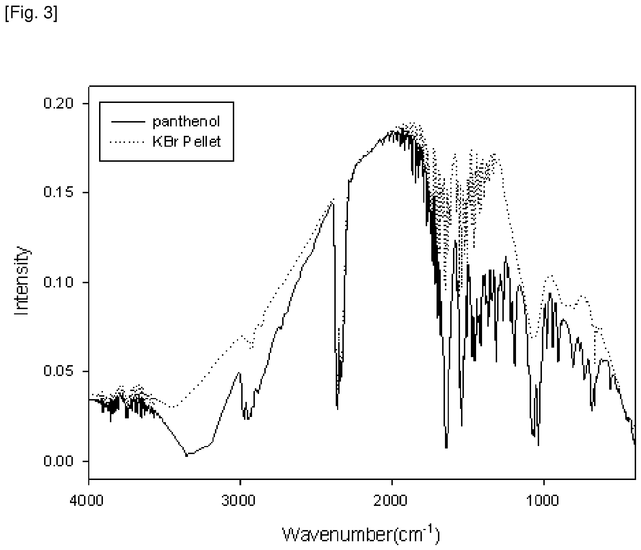

The infrared (IR) protectabilities of the compositions according to Examples 1 to 11 and Comparative Example are determined. Particularly, IR protectabilities are determined through a Fourier Transform Infrared Spectrometer, Tensor 27 (available from Bruker Co.). Samples to be analyzed are provided in the form of pellets and the test is carried out by measuring the transmission of each sample. The test results are shown in Figs. 1 to 4, wherein the wavelength range shown at the x-axis is expressed in the unit of wave number (cm⁻¹).

Referring to Figs. 1 to 4, it is said that a lower transmission in the IR range means higher IR blocking capability. It can be seen that the cosmetic compositions of Examples 1 to 11 have higher IR protectabilities, as compared to the composition of Comparative Example.

### [Test Example 2] Determination of near-IR absorbance

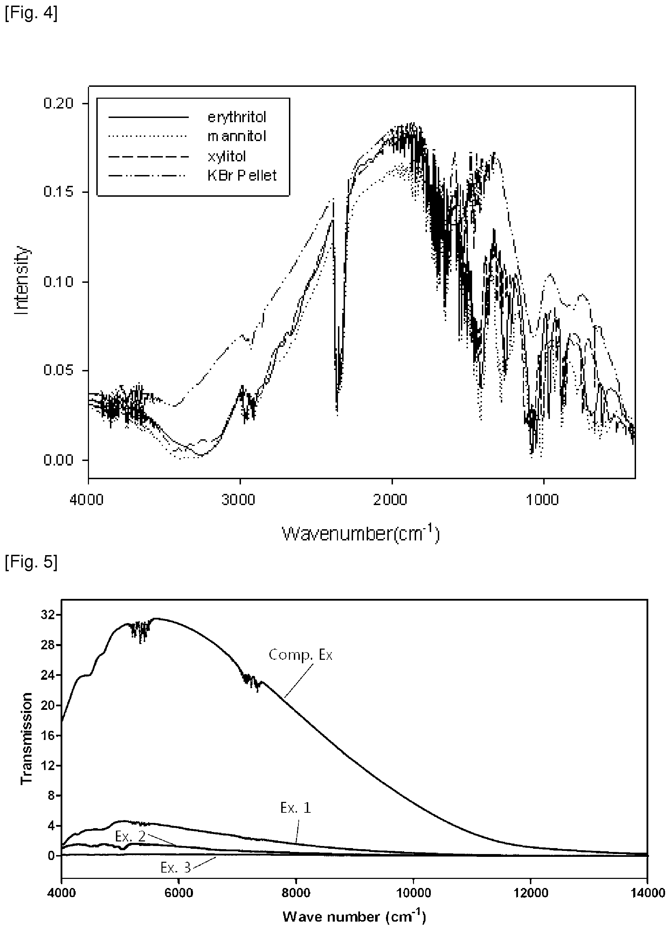

The near-IR protectabilities of the compositions according to Examples 1 to 3 and Comparative Example are determined. Particularly, near-IR protectabilities are determined through a Fourier Transform Infrared Spectrometer, Tensor 27 (available from Bruker Co.). Samples to be analyzed are provided in the form of pellets and the test is carried out by measuring the transmission of each sample. The test results are shown in Fig. 5, wherein the wavelength range shown at the x-axis is expressed in the unit of wave number (cm⁻¹).

Referring to Fig. 5, it is said that a lower transmission at the near-IR range means higher near-IR blocking capability. It can be seen that the cosmetic compositions of Examples 1 to 3 have higher near-IR protectabilities, as compared to the composition of Comparative Example. Particularly, the cosmetic compositions according to Examples 2 and 3 block most of near-IR rays in a region of 4,000-10,000 cm⁻¹.

The formulation examples will now be described. However, the cosmetic composition described herein may be formulated in various forms other than those described hereinafter. The following formulation examples are for illustrative purposes only and not intended to limit the scope of this disclosure. Formulation examples 1-4 are not according to the invention.

**[Formulation Example 1] Lotion**

| | |
|---|---|
| Inositol (Ex. 3) | 13.00 |
| L-ascorbic acid 2-phosphate magnesium salt | 1.00 |
| Water soluble collagen (1% aq. solution) | 1.00 |
| Sodium citrate | 0.10 |
| Citric acid | 0.05 |
| Licorice extract | 0.20 |
| 1,3-Butylene glycol | 3.00 |
| Deionized water | Balance |
| unit: wt% | |

**[Formulation Example 2] Cream**

| | |
|---|---|
| Niacinamide (Ex. 2) | 1.00 |
| Polyethylene glycol monostearate | 2.00 |
| Self-emulsified glycerin monostearate | 5.00 |
| Cetyl alcohol | 4.00 |
| Squalene | 6.00 |
| Tri-2-ethylhexanoic acid glyceryl ester | 6.00 |
| Spingoglycolipid | 1.00 |
| 1.3-Butylene glycol | 7.00 |
| Deionized water | Balance |
| unit: wt% | |

**[Formulation Example 3] Pack**

| | |
|---|---|
| Urea (Ex. 1) | 5.00 |
| Polyvinyl alcohol | 13.00 |
| L-ascorbic acid 2-phosphate magnesium salt | 1.00 |
| Lauroylhydroxyproline | 1.00 |
| Water soluble collagen (1% aq. solution) | 2.00 |
| 1.3-Butylene glycol | 3.00 |
| Ethanol | 5.00 |
| Deionized water | Balance |
| unit: wt% | |

**[Formulation Example 4] Essence**

| | |
|---|---|
| Inositol (Ex. 3) | 2.00 |
| Hydroxyethylene cellulose (2% aq. solution) | 12.00 |
| Xanthan gum (2% aq. solution) | 2.00 |
| 1,3-Butylene glycol | 6.00 |
| Glycerin | 4.00 |
| Sodium hyaluronate (1% aq. solution) | 5.00 |
| Deionized water | Balance |
| unit: wt% | |

## Claims

1. Non-therapeutic cosmetic use of a cosmetic composition comprising at least one amine selected from the group consisting of alanine, lysine, or salt thereof for blocking near-infrared (IR) rays to prevent skin aging,
wherein the near-IR rays have a wavelength of 780-3000 nm, and the cosmetic composition comprises at least one amine selected from the group consisting of alanine, lysine, or salt thereof in an amount of 0.001-20 wt% based on the total weight of the composition.

2. The use according to claim 1, wherein the cosmetic composition prevents thermal aging.

3. The use according to claim 1, wherein the cosmetic composition further blocks heat and IR rays, wherein the IR rays have a wavelength of 3000-25000 nm .

4. A composition comprising:
0.001-20 wt% based on the total weight of the composition of at least one amine selected from the group consisting of alanine, lysine; and an ultraviolet (UV)-blocking agent;
wherein the composition blocks heat, near-infrared (IR) rays having a wavelength of 780-3000 nm and IR rays having a wavelength of 3000-25000 nm,
for use as a medicament for blocking ultraviolet (UV), near-infrared (IR) rays, IR rays and heat.

5. The composition for use according to claim 4, wherein the composition prevents thermal aging and photo-aging simultaneously.

6. The composition for use according to claim 4, which blocks UV A and UV B.

## Patentansprüche

1. Nicht-therapeutische kosmetische Verwendung einer kosmetischen Zusammensetzung, umfassend mindestens ein Amin, ausgewählt aus der Gruppe, bestehend aus Alanin, Lysin oder einem Salz davon, zum Blockieren von Nah-Infrarotstrahlen (IR-Strahlen), um Hautalterung zu verhindern,
wobei die Nah-IR-Strahlen eine Wellenlänge von 780-3000 nm aufweisen und die kosmetische Zusammensetzung mindestens ein Amin, ausgewählt aus der Gruppe bestehend aus Alanin, Lysin oder einem Salz davon, in einer Menge von 0,001-20 Gew.-%, bezogen auf das Gesamtgewicht der Zusammensetzung, umfasst.

2. Verwendung nach Anspruch 1, wobei die kosmetische Zusammensetzung die thermische Alterung verhindert.

3. Verwendung nach Anspruch 1, wobei die kosmetische Zusammensetzung weiterhin Wärme und IR-Strahlen blockiert, wobei die IR-Strahlen eine Wellenlänge von 3000-25000 nm haben.

4. Eine Zusammensetzung bestehend aus:
0,001-20 Gew.-%, bezogen auf das Gesamtgewicht der Zusammensetzung, mindestens eines Amins, ausgewählt aus der Gruppe bestehend aus Alanin, Lysin; und ein Ultraviolett (UV)-Blockierungsmittel;
wobei die Zusammensetzung Wärme, Nah-Infrarotstrahlen (IR-Strahlen) mit einer Wellenlänge von 780-3000 nm und IR-Strahlen mit einer Wellenlänge von 3000-25000 nm blockiert,
zur Verwendung als Medikament zum Blockieren von Ultraviolett- (UV), Nahinfrarot- (IR) Strahlen, IR-Strahlen und Wärme.

5. Zusammensetzung zur Verwendung nach Anspruch 4, wobei die Zusammensetzung gleichzeitig thermische Alterung und Photoalterung verhindert.

6. Zusammensetzung zur Verwendung nach Anspruch 4, wobei die Zusammensetzung UV A und UV B blockiert.

## Revendications

1. Utilisation cosmétique non thérapeutique d'une composition cosmétique comprenant au moins une amine choisie dans le groupe constitué par l'alanine, la lysine, ou un sel de celles-ci pour bloquer des rayons proches infrarouge (IR) afin de prévenir le vieillissement de la peau,
dans laquelle les rayons proches infrarouges ont une longueur d'onde de 780 à 3000 nm, et la composition cosmétique comprend au moins une amine choisie dans le groupe constitué par l'alanine, la lysine ou un sel de celles-ci en une quantité de 0,001 à 20 % en poids sur la base du poids total de la composition.

2. Utilisation selon la revendication 1, dans laquelle la composition cosmétique prévient le vieillissement lié à la chaleur.

3. Utilisation selon la revendication 1, dans laquelle la composition cosmétique bloque en outre la chaleur et les rayons IR, dans laquelle les rayons IR ont une longueur d'onde de 3000 à 25000 nm.

4. Composition comprenant :
0,001 à 20 % en poids, par rapport au poids total de la composition, d'au moins une amine choisie dans le groupe constitué par l'alanine, la lysine ; et un agent bloquant les ultraviolets (UV) ;
dans laquelle la composition bloque la chaleur, les rayons proches infrarouge (IR) ayant une longueur d'onde de 780 à 3000 nm et les rayons IR ayant une longueur d'onde de 3000 à 25000 nm,
pour une utilisation comme médicament pour bloquer les rayons ultraviolets (UV), proches infrarouge (IR), les rayons IR et la chaleur.

5. Composition pour une utilisation selon la revendication 4, dans laquelle la composition prévient simultanément le vieillissement par la chaleur et le photovieillissement.

6. Composition pour une utilisation selon la revendication 4, qui bloque les UV A et les UV B.
